(19) Europäisches Patentamt
European Patent Office
Office européen des brevets

(11) Veröffentlichungsnummer: **0 163 905**
**B1**

(12) # EUROPÄISCHE PATENTSCHRIFT

(45) Veröffentlichungstag der Patentschrift: **25.01.89**

(51) Int. Cl.⁴: **C 07 D 307/935,** A 61 K 31/557 // C07C177/00

(21) Anmeldenummer: **85104957.7**

(22) Anmeldetag: **11.01.85**

(54) **Racemische und optisch aktive 7-Oxo-prostacyclin-Derivate, Verfahren zu deren Herstellung und diese Verbindungen enthaltende pharmazeutische Präparate.**

(30) Priorität: **13.01.84 HU 12884**

(43) Veröffentlichungstag der Anmeldung: **11.12.85 Patentblatt 85/50**

(45) Bekanntmachung des Hinweises auf die Patenterteilung: **25.01.89 Patentblatt 89/4**

(84) Benannte Vertragsstaaten: **AT BE CH DE FR GB IT LI LU NL SE**

(56) Entgegenhaltungen:
**FR-A-2 381 043**
**US-A-4 330 553**

**Die Akte enthält technische Angaben, die nach dem Eingang der Anmeldung eingereicht wurden und die nicht in dieser Patentschrift enthalten sind.**

(73) Patentinhaber: **CHINOIN Gyogyszer és Vegyészeti Termékek Gyára RT., To utca 1-5, H-1045 Budapest IV (HU)**

(72) Erfinder: **Győry, Peter, Dr., Gyakorló u. 32, H-1106 Budapest (HU)**
Erfinder: **Galambos, Géza, Dr., Lavotta u. 60, H-1104 Budapest (HU)**
Erfinder: **Kánay, Károly, Katona J. U. 61, H-2364 Ocsa (HU)**
Erfinder: **Ivanics, József, Elem u. 20, H-1045 Budapest (HU)**
Erfinder: **Dormán, György, Práter u. 50, H-1083 Budapest (HU)**
Erfinder: **Kovács, Gábor, Dr., Rónapark 4, H-1142 Budapest (HU)**
Erfinder: **Stadler, István, Dr., Népstadion ut 18, H-1143 Budapest (HU)**
Erfinder: **Virág, Sándor, Dr., Sallai I. u. 28/a, H-1136 Budapest (HU)**
Erfinder: **Tömösközi, István, Dr., Pozsonyi u. 6/a, H-1045 Budapest (HU)**
Erfinder: **Körmöczy, Péter, Dr., Uri u. 33, H-1014 Budapest (HU)**
Erfinder: **Kovács, Marianna, Dutka A. u. 63, H-1029 Budapest (HU)**

(74) Vertreter: **Lotterhos, Hans Walter, Dr.- Ing., Lichtensteinstrasse 3, D-6000 Frankfurt am Main 1 (DE)**

**EP 0 163 905 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft biologisch aktive neue Verbindungen der allgemeinen Formel I

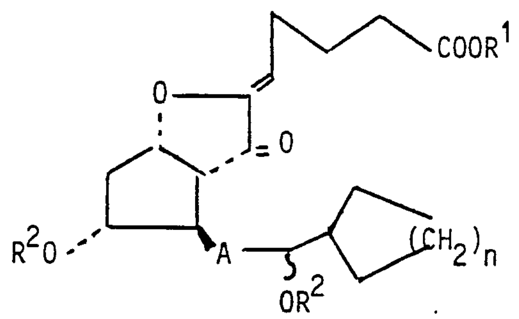

(I) ,

worin

$R^1$ Wasserstoff, geradkettiges oder verzweigtes $C_{1-4}$-Alkyl oder ein pharmakologisch anwendbares Kation,

$R^2$ Wasserstoff, $C_{1-4}$-Alkanoyl, Benzoyl, durch Halogen oder $C_{1-4}$-Alkyl substituiertes Benzoyl, Tetrahydropyranyl, Trialkylsilyl oder Alkoxy-alkyl,

A Äthylen, cis- oder trans-Vinylen oder -C≡C- und

n 2, 3 oder 4 bedeuten,

Verfahren zu deren Herstellung und diese Verbindungen als Wirkstoff enthaltende pharmazeutische Präparate.

Die Verbindungen der allgemeinen Formel I können als Racemate oder als optisch aktive Derivate vorliegen.

Das unter $R^1$ genannte pharmakologisch verträgliche Kation kann organisch oder anorganisch sein und soll in der eingesetzten Dosis keine schädlichen (toxischen) Wirkungen aufweisen. Einige Beispiele für anorganische Kationen sind: Alkalimetalle, wie z. B. Natrium oder Kalium sowie Erdalkalimetalle, wie z. B. Calcium oder Magnesium. Auch das unsubstituierte oder das durch organische Gruppen, z. B. durch Alkylgruppen, substituierte Ammoniumion ist geeignet, wobei die Substituenten des substituierten Ammoniumions noch weitere Substituenten tragen können, die die Löslichkeit und Kristallisierbarkeit der Salze günstig beeinflussen, z. B. Hydroxylgruppen oder Aminogruppen. Ein solches Kation ist das Tris-(hydroxymethyl)-ammoniumion.

Unter geradkettigem oder verzweigtem $C_{1-4}$-Alkyl ist Methyl, Äthyl, Propyl oder iso-Propyl, vorzugsweise Methyl, zu verstehen.

Bezüglich der Substituenten von $R^2$ ist

unter $C_{1-4}$-Alkanoyl eine Alkancarbonsäuregruppe aus $C_{1-4}$-Alkanen, wie z. B. Formyl, Propionyl, Butyryl, vorzugsweise Acetyl, und

unter substituiertem Benzoyl ein durch ein oder mehrere Halogene, z. B. Fluor, Chlor, Jod, Brom oder durch ein oder mehrere geradkettige oder verzweigte $C_{1-4}$-Alkyle substituiertes Benzoyl zu verstehen;

unter Trialkylsilyl ist ein aus drei geradkettigen und/oder verzweigten, gleichen oder verschiedenen $C_{1-4}$-Alkylen, die an das zentrale Siliciumatom gebunden sind, bestehendes Molekül, wie z. B. Trimethylsilyl, Triäthyl-silyl, Tripropyl-silyl oder Dimethyl-i-propyl-silyl, vorzugsweise Dimethyl-tert.butyl-silyl und

unter Alkoxy-alkyl ist die Gruppe

$R - O - CH_2-$,

worin

R geradkettiges oder verzweigtes $C_{1-4}$-Alkyl, vorzugsweise Methyl, bedeutet, zu verstehen.

Zur Herstellung der 7-Oxo-$PGI_2$-Derivate der allgemeinen Formel I kann man entweder

a) eine Verbindung der allgemeinen Formel IV

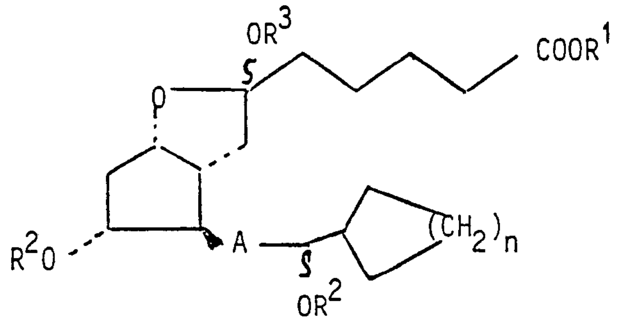

(IV),

worin
$R^1$, $R^2$, A und n die oben angegebene Bedeutung haben und
$R^3$ Wasserstoff, geradkettiges oder verzweigtes $C_{1-4}$-Alkyl bedeutet, oxydieren und die erhaltene 7-Oxo-Verbindung der allgemeinen Formel II

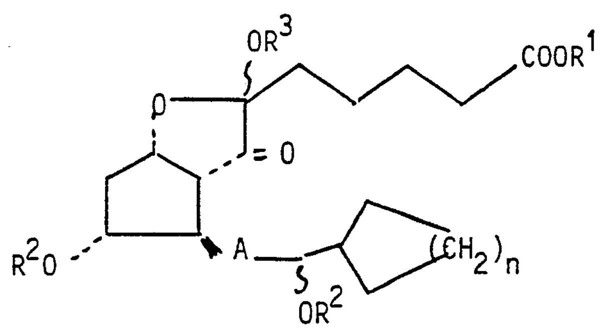

(II),

worin
$R^1$, $R^2$, $R^3$, A und n die oben angegebene Bedeutung haben,
erhitzen oder
b) eine Verbindung der allgemeinen Formel III

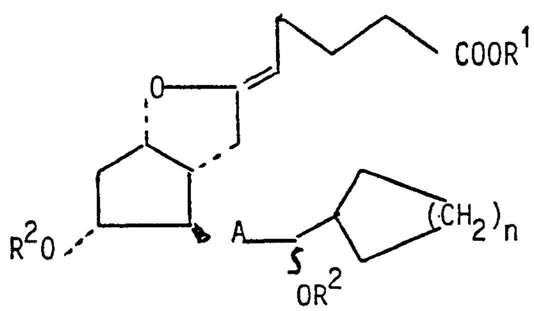

(III),

worin
$R^1$, $R^2$, A und n die oben angegebene Bedeutung haben,
oxydieren, wobei in den nach Variante a) oder Variante b) erhaltenen 7-Oxo-$PGI_2$-Derivaten der Formel I gewünschtenfalls die Schutzgruppen der 11- und/oder 15-Hydroxylgruppen und/oder der Carboxylgruppe entfernt und/oder die 7-Oxo-$PGI_2$-Derivate in pharmakologisch verträgliche Salze übergeführt werden können.

Die Verbindungen der allgemeinen Formel I sind stabile Prostacyclin-Derivate, die selektive biologische Wirkungen aufweisen.

Der in den Säugetieren in größerem Umfang vorkommende Arachidonsäure-Metabolit, Prostacyclin-$PGI_2$, wurde 1976 entdeckt.

Die Verbindung hat zahlreiche, therapeutisch nutzbare biologische Wirkungen. Sie hemmt die Aggregation der Thrombozyten, zeigt eine fibrinolytische Wirkung, erweitert die Luftwege und Blutgefäße und setzt die Sekretion der Magensäure herab. Außerdem weist sie in verschiedenen Organen, wie z. B. im Magen, in der Leber, im Herz und in den Nieren eine sogenannte cytoprotektive Wirkung auf, d. h. sie eliminiert oder heilt die destruktiven Folgeerscheinungen der schädlichen Wirkungen, die in verschiedenen Organen auftreten können.

Einer breiteren Anwendung des $PGI_2$ steht jedoch einerseits dessen chemische und biologische Instabilität

entgegen, die sich in der sehr kurzen biologischen Halbzeit äußert und die zur Folge hat, daß die Applikation nur auf sehr spezielle Weise, z. B. durch Infusion erfolgen kann. Andererseits stehen einer breiteren Anwendung die gegebenenfalls beim $PGI_2$ auftretenden unerwünschten Nebenwirkungen entgegen, die als Folge des komplexen biologischen Wirkungsspektrums neben der gewünschten Wirkung auftreten.

Die Herstellung von 7-Oxo-$PGI_2$-Derivaten, die eine größere Stabilität als das natürliche $PGI_2$ haben, ist in der US-PS-4 330 553 beschrieben. Die darin genannten Verbindungen sind chemisch stabiler als $PGI_2$ - sie können z. B. in wäßriger Lösung gelagert werden - und sie haben das gleiche Wirkungsspektrum wie $PGI_2$ (J. Med. Chem. 25, (1982) 105).

Die Herstellung ännlicher 7-Oxo-$PGI_2$-Derivate ist in der BE-PS-890 390 beschrieben. Die aus diesen Patentschriften bekannten 7-Oxo-$PGI_2$-Derivate haben die gemeinsame Eigenschaft, daß ihre biologische Wirkung ähnlich wie beim $PGI_2$ nicht genügend selektiv ist.

Die Herstellung von 15-Cycloalkyl-16,17,18,19,20-pentanor-$PGI_2$-Derivaten ist aus der FR-A-2 381 043 bekannt. Die in dieser Patentschrift beschriebenen Verbindungen weisen aber eine ungenügende chemische Stabilität auf.

Die neuen 7-Oxo-$PGI_2$-Derivate der Erfindung haben das gleiche Wirkungsspektrum wie $PGI_2$. Es wurde aber überraschenderweise gefunden, daß die neuen Verbindungen der Erfindung ihre biologische Wirkung selektiver ausüben, als die 7-Oxo-$PGI_2$-Derivate des Standes der Technik, mit denen sie in der Stabilität etwa übereinstimmen.

Die Herstellung der 7-Oxo-$PGI_2$-Derivate der allgemeinen Formel I erfolgt gemäß Erfindung zweckmäßig in folgender Weise:

a) Eine Verbindung der allgemeinen Formel IV wird in einem Wasser enthaltenden Lösungsmittel, vorzugsweise Dioxan oder Dimethoxyäthan, mit 1,1 - 10, vorzugsweise 1,3 - 1,5 Äquivalenten Selendioxyd bei 20 - 200, vorzugsweise 50 - 100° C, oxydiert.

Die so erhaltene 7-Oxo-Verbindung der allgemeinen Formel II wird in Gegenwart eines Säurekatalysators in organischen Lösungsmitteln - vorzugsweise in einem aromatischen Lösungsmittel, wie Benzol oder Toluol - und/oder in halogenierten Lösungsmitteln, wie chlorierten Kohlenwasserstoffen, vorzugsweise Chloroform, und/oder in dipolaren aprotischen Lösungsmitteln, wie z. B. Dimethylsulfoxyd, oder in Abwesenheit von Lösungsmitteln erhitzt, wobei man ein 7-Oxo-$PGI_2$-Derivat der allgemeinen Formel I erhält. Als Säurekatalysator wird p-Toluolsulfonsäure oder Schwefelsäure bevorzugt. Die 7-Oxo-Verbindung der allgemeinen Formel II kann bei 50 - 200, vorzugsweise 60 - 90° C, mit Wasserabscheidung oder ohne erhitzt werden.

Zweckmäßig wird so vorgegangen, daß man das Erhitzen in Gegenwart von p-Toluolsulfonsäure in Benzol oder Toluol bei 60° C unter Verwendung eines Wasserabscheiders durchführt.

Die Verbindungen der allgemeinen Formel IV sind entweder aus der Literatur bekannt oder sie können nach literaturbekannten Methoden hergestellt werden (z. B. HU-PS-180 442 (Patentanmeldung Nr. CI-1901)).

b) Nach einer anderen vorteilhaften Ausführungsform der Erfindung wird eine Verbindung der allgemeinen Formel III zum entsprechenden 7-Oxo-$PGI_2$-Derivat der allgemeinen Formel I oxydiert, wobei man die Oxydation bei 20 - 200° C mit 1,1 - 10 Äquivalenten Selendioxyd in wasserfreiem organischem Lösungsmittel, zweckmäßig in einem Äther, vorzugsweise in Dioxan oder Dimethoxyäthan durchführt. Nach einer besonders vorteilhaften Ausführungsform der Erfindung wird die Reaktion in Dioxan mit 1,3 - 1,5 Äquivalenten Selendioxyd bei 80 - 90° C durchgeführt.

Die Verbindungen der allgemeinen Formel III sind entweder aus der Literatur bekannt oder sie können nach literaturbekannten Methoden hergestellt werden (R. F. Newton et al., Synthesis 1984, 449).

Die Hydroxylgruppen in den 11- und 15-Stellungen der Ausgangsverbindungen der allgemeinen Formel III und IV können durch die in der Prostacyclinchemie bekannten Schutzgruppen blockiert werden. Einige Beispiele für solche Gruppen sind: verschiedene Acylgruppen, Trialkylsilylgruppen, Alkoxyalkylgruppen sowie die Tetrahydropyranylgruppe. Nähere Einzelheiten über diese Gruppen sind weiter oben angegeben. Die Einführung der Schutzgruppen kann mittels literaturbekannter Methoden erfolgen, dies kann zur Erzielung besserer Ausbeuten wichtig sein.

Diese Schutzgruppen können gewünschtenfalls durch literaturbekannte Methoden aus den 7-Oxo-$PGI_2$-Derivaten der allgemeinen Formel I abgespalten werden. Die Alkoxyalkylgruppen sowie die Tetrahydropyranylgruppe können durch saure Hydrolyse und die Trialkylsilylgruppen können entweder mit Säure oder mit Kaliumfluorid entfernt werden. Die Acylgruppen werden in basischem Medium hydrolysiert.

Die Vertreter der 7-Oxo-$PGI_2$-Derivate der allgemeinen Formel I - in denen $R^1$ geradkettiges oder verzweigtes $C_{1-4}$-Alkyl ist - können in an sich bekannter Weise in ein pharmakologisch verträgliches Kation enthaltendes Salz übergeführt werden. Zu dem Zweck wird die Estergruppe unter basischen Bedingungen abgespalten. Als Basen kommen wäßrige oder wasserfreie alkoholische Lösungen von Alkali- und Erdalkali-hydroxyden oder -alkoxyden in Frage. Als Base wird vorzugsweise Natriummethoxyd und als Lösungsmittel wird vorzugsweise wasserfreies Methanol eingesetzt. Aus den wäßrigen Lösungen der so erhaltenen Salze können die Carbonsäuren durch vorsichtiges Ansäuern freigesetzt werden, dann in organischen Lösungsmitteln gelöst und mit aliphatischen oder aromatischen Aminen in weitere Salze, z. B. das Trimethoniumsalz, übergeführt werden.

Ein Vertreter der Verbindungen der allgemeinen Formel I ist das Natriumsalz von 7-Oxo-16,17,18,19,20-pentanor-15-cyclopentyl-$PGI_2$. Die selektive biologische Wirkung der Verbindungen der Erfindung kann durch die gleichzeitige Untersuchung der hypotensiven und der anti-aggregativen Wirkung festgestellt werden. Die Antiaggregationswirkung wurde in vitro (Nature, 214, (1962) 927) bei an Thrombocyten angereichertem

humanen Plasma in einer mit 2 µM ADP induzierten Aggregation gemessen, wobei die hämodynamische Wirkung aus der den Blutdruck senkenden Wirkung der durch i.v. bolus Injektion verabreichten Verbindung an anästhetisierten Katzen mit offener Brust bestimmt wurde.

| | antiaggregative Wirkung $IC_{50}$ ng/ml | relative Effektivität $H^1$ | hypotensive Wirkung $ED_{50}$ µg/kg | relative Effektivität $H^2$ | $H^1$/H2 |
|---|---|---|---|---|---|
| $PGI_2$-Na | 1 | 1 | 0,16 | 1 | 1 |
| 7-Oxo-$PGI_2$-Na | 15 | 0,066 | 6,5 | 0,024 | 2,75 |
| 7-Oxo-16,17,18,19, 20-pentanor-15-cyclopentyl-$PGI_2$-Na | 1,6 | 0,625 | 21,2 | 0,0075 | 83,3 |

Die Tabelle zeigt, daß das 7-Oxo-15-cyclopentyl-$PGI_2$-Derivat der Erfindung etwa 100-mal selektiver als Prostacyclin ist, daß es aber auch wesentlich selektiver als das in der US-PS-4 330 533 beschriebene 7-Oxo-$PGI_2$-Na ist.

Ein besonderer Vorteil, den 7-Oxo-$PGI_2$-Derivate der allgemeinen Formel I gegenüber $PGI_2$ aufweisen, besteht darin, daß sie oral anwendbar sind.

Auf Grund ihrer biologischen Aktivität können die Verbindungen der Erfindung als Wirkstoffe für pharmazeutische Präparate benutzt werden.

Diese Präparate können zur Vorbeugung bzw. Heilung von Krankheiten, wie z. B. peripheren Gefäßerkrankungen (Artherosclerosis obliterans, Bürger-Krankheit. Diabeticus angipethia) verwendet werden.

Weitere Anwendungsmöglichkeiten sind Verbesserung des Kreislaufes der Extremitäten, Reduktion der Größe des Infarktes beim Herzinfarkt und Herabsetzung der Mortalität. Die Präparate der Erfindung sind auch geeignet, um Anzahl und Stärke der Anfälle bei verschiedenen Typen von Angina-Krankheiten herabzusetzen, und um verschiedene Kreislauf-Krankheiten, z. B. Pulmonal-Hypertension und Herzinsuffizienz zu heilen. Sie stellen wertvolle Mittel zur Prävention und Heilung von Hirn-Ischämie dar, sie können außerdem zur Heilung von Asthma, von Erkrankungen des gastrointestinalen Systems (z. B. Ulcus), der Leber und des Pankreas eingesetzt werden und man kann mit den Präparaten der Erfindung den Thrombocytenverlust bei extrakorporalem Kreislauf (Nieren-Chemodialyse, Herz-Lungenmaschine) allein oder in Kombination mit Heparin verhindern. Ein neueres Anwendungsgebiet ist die Hemmung der Metastasen bei Tumorerkrankungen.

Ein Vorteil der Präparate der Erfindung besteht darin, daß sie eine Behandlung durch intravenöse, subcutane, intramusculäre und orale (gastrointestinale) Applikation ermöglichen. Die erforderliche Menge beträgt 0,0001 - 10 mg/kg Körpergewicht. Die genaue Dosis hängt davon ab, wie schwer die Krankheit ist, wie schnell das Arzneimittel vom Organismus resorbiert wird und wie hoch die individuelle Empfindlichkeit und die Reaktionsfähigkeit des zu behandelnden Organs ist. Die benötigte Dosis und die bevorzugte Verabreichungsmethode ist vom Fachmann auf Grund seiner Fachkenntnisse leicht festzustellen.

Bei der Herstellung der pharmazeutischen Präparate der Erfindung können die üblichen Streckmittel, Verdünnungsmittel, Mittel, die den Geschmack und das Aroma verbessern, die die Formulierung erleichtern, mit denen der pH und der osmotische Druck eingestellt wird, die die Resorption erleichtern. Stabilisierungsmittel, etc. sowie weitere Hilfsstoffe verwendet werden. Die Präparate der Erfindung können fest (Tabletten, Kapseln, Dragées, Pulver, Pillen), flüssig (Infusionslösungen, Inhalationslösungen, Injektionslösungen, Umschläge, Tropfen, Löffelarznei) oder semi-flüssig (Cremes, Salben, Balsam, Suppositorien usw.) sein. Die Wirkstoffe der Erfindung können auch mit anderen Wirkstoffen kombiniert werden.

Die Erfindung soll durch die nachfolgenden Beispiele näher erläutert werden.

**Beispiel 1**

6-Hydroxy-7-oxo-11,15-diacetyl-16,17,18,19,20-pentanor-15-cyclopentyl-$PGI_1$-methylester.

605 mg (1,25 mMol) 6-Methoxy-11,15-diacetyl-16,17,18,19,20-pentanor-15-cyclopentyl-$PGI_1$-methylester löst man in 6 ml Dioxan und 0,5 ml Wasser, gibt 210 mg (1,89 mMol) Selendioxyd zu und rührt das Reaktionsgemisch bei 60°C 2 Stunden. Nach Beendigung der Reaktion wird das Reaktionsgemisch filtriert und das Lösungsmittel im Vacuum entfernt. Der Rückstand wird mit 50 ml Äthylacetat verdünnt, 2-mal mit je 10 ml Wasser und dann mit einer 5-%-igen Natriumbicarbonatlösung so lange gewaschen bis der pH-Wert 7,5 beträgt. Schließlich wird er mit gesättigter Salzlösung gewaschen und über Magnesiumsulfat getrocknet. Das so erhaltene Rohprodukt wird auf 200 g Reanal Kieselgel G Adsorbens der Kurz-Kolonnen-Chromatographie unterworfen und mit einer 50-%-igen Hexan-Äthylacetat-Mischung eluiert, wobei man 380 mg 6-Hydroxy-7-oxo-11,15-diacetyl-16,17,18,19,20-pentanor-15-cyclopentyl-$PGI_1$-methylester in Form eines roten Öls erhält.

Dünnschichtchromatographie: $R_f$: 0,18 (1 Hexan - 1 Äthylacetat)

IR: ($cm^{-1}$, Film): 3400, 2950, 1740, 1150.

**Beispiel 2**

7-Oxo-11,15-diacetyl-16,17,18,19,20-pentanor-15-cyclopentyl-$PGI_2$-methylester.

380 mg (0,79 mMol) 6-Hydroxy-7-oxo-11,15-diacetyl-16,17,18,19,20-pentanor-15-cyclopentyl-$PGI_1$-methylester löst man in 30 ml Benzol, gibt zu der Lösung 3 mg p-Toluolsulfonsäure und rührt das Gemisch zunächst 16 Minuten bei Raumtemperatur und dann 3 Stunden bei 60° C. Während des Erhitzens destillieren 5 ml Benzol in den Wasserabscheider über.

Nach Beendigung der Reaktion kühlt man das Reaktionsgemisch auf Raumtemperatur und rührt das Gemisch 2 Stunden mit 0,6 g neutralem Aluminiumoxyd. Das Aluminiumoxyd filtriert man ab und engt das Filtrat teilweise ein. Das so erhaltene Produkt wird der Kurz-Kolonnen-Chromatographie auf einem Kieselgel G Adsorbens unterworfen und mit einer 2 : 1 Mischung von Hexan und Äthylacetat eluiert. Man isoliert 199 mg 7-Oxo-11,15-diacetyl-16,17,18,19,20-pentanor-15-cyclopentyl-$PGI_2$-methylester in Form eines farblosen Öls.

$R_f$ = 0,43 (1 Hexan - 1 Äthylacetat)

IR: ($cm^{-1}$, Film): 2950, 1745, 1715, 1650, 1160

UV $\lambda_{max}$ (nm) = 287 lg $\varepsilon$ = 3,96

**Beispiel 3**

7-Oxo-16,17,18,19,20-pentanor-15-cyclopentyl-$PGI_2$-methylester.

198 mg (0,43 mMol) 7-Oxo-11,15-diacetyl-16,17,18,19,20-pentanor-15-cyclopentyl-$PGI_2$-methylester löst man in 30 ml abs. Methanol, gibt zu der Lösung 0,25 ml einer 1M Natrium-methoxydlösung in Methanol und rührt das Reaktionsgemisch über Nacht bei Raumtemperatur. Das nach dem Entfernen des Methanols bei 0°C i.V. erhaltene Produkt wird in einem Gemisch von 50 ml Äther und 7 ml Wasser bei 0°C gelöst. Die nach der Phasentrennung erhaltene Ätherlösung wird zunächst mit 10 ml gesättigter Salzlösung gewaschen, dann mit 0,5 g neutralem Aluminiumoxyd und 0,2 g Aktivkohle gerührt, filtriert und schließlich in Gegenwart von Triäthylamin über Natriumsulfat getrocknet. Nach dem Einengen erhält man 135 mg 7-Oxo-16,17,18,19,20-pentanor-15-cyclopentyl-$PGI_2$-methylester in reiner Form als farbloses Öl.

$R_f$ = 0,36 (1 Hexan - 1 Aceton)

UV: $\lambda_{max}$ = 287 nm. 1g$\varepsilon$ = 3,856

**Beispiel 4**

7-Oxo-16,17,18,19,20-pentanor-15-cyclopentyl-$PGI_2$-natriumsalz.

220 mg (0,63 mMol) 7-Oxo-16,17,18,19,20-pentanor-cyclopentyl-$PGI_2$-methylester löst man in 10 ml Methanol, gibt 2 ml 1M wäßrige Natriumhydroxydlösung zu und rührt das Gemisch 3 Stunden bei 40° C. Nach Entfernen des Methanols i.V. löst man den Rückstand in 20 ml Wasser, schüttelt die wäßrige Lösung 2-mal mit je 5 ml Äther aus, säuert die wäßrige Phase mit 1 n Natrium-bisulfatlösung bei 0° C auf pH 6 an und schüttelt sie 2-mal mit je 10 ml Äthylacetat aus. Die wäßrige Phase wird mit weiterer Natrium-bisulfatlösung bei 0°C auf pH 4 angesäuert und 2-mal mit je 20 ml Äthylacetat gewaschen.

Die Äthylacetat-haltigen organischen Phasen werden vereinigt und 2-mal mit je 5 ml gesättigter Salzlösung gewaschen. Die erhaltene Lösung rührt man mit 0,6 g neutralem Aluminiumoxyd und 0,2 g Aktivkohle 10 Minuten und filtriert sie. Danach gibt man zu dieser Lösung 20 ml Wasser und neutralisiert den pH mit 0,1 n Natriumhydroxydlösung auf einen Wert von 7,2 - 7,3. Die beiden Phasen werden getrennt, man schüttelt die organische Phase mit 10 ml Wasser aus, engt die vereinigten wäßrigen Phasen ein und trocknet sie. Man erhält 190 mg des 7-Oxo-16,17,18,19,20-pentanor-15-cyclopentyl-$PGI_2$-Natriumsalz. Um die wäßrige Lösung der Titelverbindung weiter einzusetzen, braucht die wäßrige Lösung nicht eingeengt zu werden, die Lösung kann unmittelbar weiter verwendet werden.

Dünnschichtchromatographie: in Säureform analysiert

$R_f$ = 0,30

(Laufmittel: 20 Benzol, 10 Dioxan, 1 Essigsäure)

IR: ($cm^{-1}$, KBr): 3500 - 3200, 2940, 2850, 1720, 1650, 1615.

**Beispiel 5**

7-Oxo-16,17,18,19,20-pentanor-15-cyclopentyl-$PGI_2$-natriumsalz.

340 mg (0,74 mMol) 7-Oxo-11,15-diacetyl-16,17,18,19,20-pentanor-15-cyclopentyl-$PGI_2$-methylester löst man in 50 ml Methanol, gibt zu der Lösung 0,5 ml einer 1M methanolischen Natrium-methoxydlösung und rührt das Reaktionsgemisch 10 Stunden bei Raumtemperatur. Danach entfernt man teilweise das Methanol (Total-Volumen: ca. 20 ml), gibt dann 5 ml einer 1 n Natriumhydroxydlösung zu und rührt das Gemisch bei 40°C 3

Stunden. Dann wird das Methanol i.V. abdestilliert. Die weitere Aufarbeitung des Reaktionsgemisches erfolgt in der im vorhergehenden Beispiel beschriebenen Weise.

**Beispiel 6**

7-Oxo-11,15-diacetyl-16,17,18,19,20-pentanol-15-cyclohexyl-$PGI_2$-methylester.

Zu einer Lösung von 1010 mg (2,25 mMol) 11,15-Diacetyl-16,17,18,19,20-pentanor-15-cyclohexyl-$PGI_2$-methylester in 20 ml wasserfreiem Dioxan gibt man 374 mg (3,4 mMol) Selendioxyd und rührt das Reaktionsgemisch 2,5 Stunden bei 85°C unter Argon-Atmosphäre. Nach Beendigung der Reaktion gibt man zum Reaktionsgemisch bei Raumtemperatur 1 g neutrales Aluminiumoxyd und rührt das Gemisch 15 Minuten. Nach Filtrieren des Gemisches wird es teilweise eingeengt und durch Kurz-Kolonnen-Chromatographie gereinigt. Als Adsorbens verwendet man 200 g Kieselgel G und als Elutionsmittel ein 1 : 1 Gemisch von Hexan und Äthylacetat. Man erhält 280 mg des 7-Oxo-11,15-diacetyl-16,17,18,19,20-pentanor-15-cyclohexyl-$PGI_2$-methylesters in Form eines farblosen Öls.

$R_f$ = 0,40 (Hexan-Äthylacetat 1 : 1)

IR: ($cm^{-1}$, Film) = 2950, 2860, 1740, 1715, 1650.

**Beispiel 7**

7-Oxo-16,17,18,19,20-pentanor-15-cyclohexyl-$PGI_2$-methylester.

Zu einer Lösung von 220 mg (0,46 mMol) 7-Oxo-11,15-diacetyl-16,17,18,19,20-pentanor-15-cyclohexyl-$PGI_2$-methylester in 30 ml abs. Methanol gibt man 0,25 ml einer 1 M methanolischen Natriummethoxydlösung und rührt das Reaktionsgemisch über Nacht bei Raumtemperatur unter Argon-Atmosphäre. Das nach dem Entfernen des Methanols i.V. erhaltene Produkt wird in einem Gemisch von 50 ml Äther und 7 ml Wasser bei 0°C gelöst, und die nach der Phasentrennung erhaltene Ätherlösung wird mit 10 ml gesättigter Salzlösung gewaschen, sodann mit 0,5 g neutralem Aluminiumoxyd und 0,2 g Aktivkohle 10 Minuten gerührt und schließlich über Natriumsulfat getrocknet. Durch Einengen erhält man 148 mg des 7-Oxo-16,17,18,19,20-pentanor-15-cyclohexyl-$PGI_2$-methylesters in Form eines farblosen Öls.

$R_f$ = 0,34 (1 Hexan - 1 Aceton)

UV: $\lambda_{max}$ = 289 nm, lg $\varepsilon$ = 3,903

**Beispiel 8**

7-Oxo-16,17,18,19,20-pentanor-15-cyclopentyl-$PGI_2$-Tris-(hydroxymethyl)-amino-methan-salz.

540 mg (1,35 mMol) des Natriumsalzes des 7-Oxo-16,17,18,19,20-pentanor-15-cyclopentyl-$PGI_2$ werden in 10 ml Wasser bei 0°C mit 1 n Natriumbisulfatlösung auf pH 4 angesäuert und 2-mal mit je 20 ml Äthylacetat ausgeschüttelt. Die vereinigten organischen Phasen werden mit gesättigter Salzlösung ausgeschüttelt und über Natriumsulfat getrocknet. Das Natriumsulfat filtriert man ab, gibt dann 175 mg (1,45 mMol) Tris-(hydroxymethyl)-amino-methan zur Lösung, rohrt das Gemisch 2 Stunden bei 40°C und läßt es 12 Stunden stehen. Danach engt man das Reaktionsgemisch ein, wobei man 510 mg 7-Oxo-16,17,18,19,20-pentanor-15-cyclopentyl-$PGI_2$-Tris-(hydroxymethyl)-amino-methan-salz erhält.

Dünnschichtchromatographie: in Säureform wie in dem vorhergehenden Beispiel beschrieben.

UV (EtOH): $\lambda_{max}$ = 288 nm, lg $\varepsilon$ = 3,965

**Beispiel 9**

6-Hydroxy-7-oxo-11,15-diacetyl-13,14-didehydro-16,17,18,19,20-pentanor-15-cyclohexyl-$PGI_1$-methylester.

Zu einer Lösung von 1850 mg (3,76 mMol) 6-Methoxy-11,15,-diacetyl-13,14-didehydro-16,17,18,19,20-pentanor-15-cyclohexyl-$PGI_1$-methylester in 15 ml Dioxan und 2 ml Wasser gibt man 650 mg (5,86 mMol) Selendioxyd, rührt das Reaktionsgemisch bei 60°C 2 Stunden, filtriert es, entfernt das Dioxan unter vermindertem Druck und verdünnt den Rückstand mit 200 ml Äthylacetat. Die Lösung wird nacheinander 2-mal mit je 50 ml Wasser und 5-%-iger Natrium-bicarbonatlösung so lange gewaschen, bis der pH-Wert 7,5 - 8 erreicht, danach wird sie mit gesättigter Salzlösung gewaschen und über Magnesiumsulfat getrocknet. 790 mg 6-Hydroxy-7-oxo-11,15-diacetyl-13,14-didehydro-16,17,18,19,20-pentanor-15-cyclohexyl-$PGI_1$-methylester erhält man durch Kurz-Kolonnen-Chromatographie (500 g Kieselgel G, 1 : 1 Gemisch von Hexan und Äthylacetat als Elutionsmittel) in Form eines roten Öls.

Dünnschichtchromatographie:

$R_f$ = 0,20 (1 Hexan - 1 Äthylacetat)
IR: ($cm^{-1}$, Film): 3400, 2950, 2230, 1740, 1160.

**Beispiel 10**

7-Oxo-11,15-diacetyl-13,14-didehydro-16,17,18,19,20-pentanor-15-cyclohexyl-$PGI_2$-methylester.

Zu einer Lösung von 750 mg (1,52 mMol) 6-Hydroxy-7-oxo-11,15-diacetyl-13,14-didehydro-16,17,18,19,20-pentanor-15-cyclohexyl-$PGI_1$-methylester in 30 ml Benzol gibt man 5 ml p-Toluolsulfonsäure und rührt das Gemisch zunächst 15 Minuten bei Raumtemperatur und danach 3 Stunden bei 60 - 65° C, wobei das Wasser des Lösungsmittels langsam abdestilliert wird. Während der Reaktion werden 10 ml Benzol abdestilliert. Das auf Raumtemperatur abgekühlte Gemisch rührt man mit 1 g neutralem Aluminiumoxyd 3 Stunden, entfernt danach das Aluminiumoxyd durch Filtration und reinigt das erhaltene Reaktionsgemisch durch Kurz-Kolonnen-Chromatographie (100 g Kieselgel G Silicagel, Elutionsmittel: 7 Hexan - 1 Äthylacetat). Man erhält 286 mg 7-Oxo-11,15-diacetyl-13,14-didehydro-16,17,18,19,20-pentanor-15-cyclohexyl-$PGI_2$-methylester in Form eines farblosen Öls.

Dünnschichtchromatographie:
$R_f$ = 0,56 (1 Hexan - 1 Äthylacetat)
UV (EtOH): = $\lambda_{max}$ 285 nm, lg ε = 4,01

**Beispiel 11**

7-Oxo-13,14-didehydro-16,17,18,19,20-pentanor-15-cyclohexyl-$PGI_2$-methylester.

Zu einer Lösung von 250 mg (0,53 mMol) 7-Oxo-13,14-didehydro-11,15-diacetyl-16,17,18,19,20-pentanor-15-cyclohexyl-$PGI_2$-methylester in 50 ml abs. Methanol gibt man 1 ml (1 mMol in 1 M Methanol) Natriummethoxydlösung, läßt das Reaktionsgemisch über Nacht stehen und entfernt das Lösungsmittel bei vermindertem Druck bei 0° C. Den Rückstand löst man in einem Gemisch von 50 ml Äther und 5 ml Wasser, wäscht die abgetrennte organische Phase mit 10 ml gesättigter Salzsäurelösung, rührt sie mit 0,5 g neutralem Aluminiumoxyd und 0,5 g Aktivkohle 10 Minuten, filtriert sie und trocknet sie in Gegenwart von Triäthylamin über Natriumsulfat. Nach dem Einengen erhält man 143 mg 7-Oxo-13,14-didehydro-16,17,18,19,20-pentanor-15-cyclohexyl-$PGI_2$-methylester in Form eines farblosen Öls.

Dünnschichtchromatographie:
$R_f$ = 0,21 (1 Hexan - 1 Äthylacetat)
UV (EtOH): $\lambda_{max}$ = 287 nm, lg ε = 3,98

**Beispiel 12**

7-Oxo-13,14-didehydro-15,17,18,19,20-pentanor-15-cyclohexyl-$PGI_2$-natriumsalz.

Zu einer Lösung von 113 mg (0,31 mMol) 7-Oxo-13,14-didehydro-16,17,18,19,20-pentanor-15-cyclohexyl-$PGI_2$-methylester in 7 ml Methanol und 3 ml Wasser gibt man 2 ml (2 mMol, in 1 M Wasser) Natriumhydroxydlösung, rührt das Gemisch bei Raumtemperatur 3 Stunden, entfernt das Methanol bei vermindertem Druck und gibt zum Rückstand 10 ml destilliertes Wasser. Die wäßrige Lösung wird 2-mal mit je 5 ml Äther gewaschen, auf 0° C gekühlt, mit etwa 2 ml kaltem Natriumbisulfat (in 1 M Wasser) auf pH 5 - 6 angesäuert und 2-mal mit 10 ml Äthylacetat extrahiert. Die so erhaltene wäßrige Phase säuert man mit 0,1 M Natriumbisulfatlösung weiter (auf pH 3 - 4) an und wäscht sie 2-mal mit je 20 ml Äthylacetat. Die vereinigten Äthylacetatphasen werden 2-mal mit je 10 ml gesättigter Salzlösung gewaschen, mit 0,5 g neutralem Aluminiumoxyd und mit 0,2 g Aktivkohle gerührt und filtriert. Zum Filtrat gibt man 20 ml Wasser und stellt den pH-Wert mit 0,1 n Natriumhydroxydlösung auf 7,4 - 7,6 ein. Die organische Phase schüttelt man mit 10 ml Wasser aus und engt die vereinigten wäßrigen Phasen ein. Man erhält 78 mg 7-Oxo-13,14-didehydro-16,17,18,19,20-pentanor-15-cyclohexyl-$PGI_2$-Natriumsalz.

Dünnschichtchromatographie: Das Produkt wird in Form von Säure analysiert.
$R_f$ = 0,30 (20 Benzol, 10 Dioxan, 1 Essigsäure)
IR (KBr, $cm^{-1}$): 3300, 2950, 2220, 1720, 1645.

**Patentansprüche** für die Vertragsstaaten: BE, CH, DE, FR, GB, IT, LI, LU, NL, SE

1. Racemische und optische aktive 7-Oxo-prostacyclin-Derivate der allgemeinen Formel I

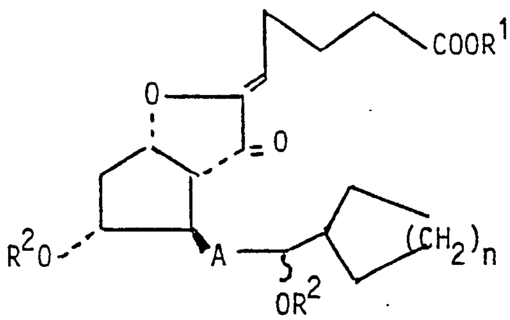

(I) ,

worin
$R^1$ Wasserstoff, ein geradkettiges oder verzweigtes $C_{1-4}$-Alkyl oder ein pharmakologisch anwendbares Kation,
$R^2$ Wasserstoff, $C_{1-4}$-Alkanoyl, Benzoyl, durch Halogen oder $C_{1-4}$-Alkyl substituiertes Benzoyl, Tetrahydropyranyl, Trialkylsilyl oder Alkoxy-alkyl,
A Äthylen, cis- oder trans-Vinylen oder -C≡C- und
n 2, 3 oder 4 bedeuten.

2. 7-Oxo-11,15-diacetyl-16,17,18,19,20-pentanor-15-cyclopentyl-$PGI_2$-methylester.

3. Methylester, Natriumsalz oder Tris-(hydroxymethyl)-amino-methansalz des 7-Oxo-16,17,18,19,20-pentanor-15-cyclopentyl-$PGI_2$.

4. 7-Oxo-11,15-diacetyl-16,17,18,19,20-pentanor-15-cyclohexyl-$PGI_2$-methylester.

5. 7-Oxo-16,17,18,19,20-pentanor-15-cyclohexyl-$PGI_2$-methylester.

6. Verfahren zur Herstellung von racemischen und optisch aktiven 7-Oxo-prostacyclin-Derivaten der allgemeinen Formel I

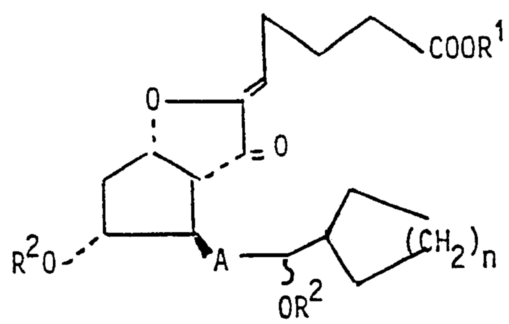

(I) ,

worin
$R^1$ Wasserstoff, ein geradkettiges oder verzweigtes $C_{1-4}$-Alkyl oder ein pharmakologisch anwendbares Kation,
$R^2$ Wasserstoff, $C_{1-4}$-Alkanoyl, Benzoyl, durch Halogen oder $C_{1-4}$-Alkyl substituiertes Benzoyl, Tetrahydropyranyl, Trialkylsilyl oder Alkoxy-alkyl,
A Äthylen, cis- oder trans-Vinylen oder -C≡C- und
n 2, 3 oder 4 bedeuten,
dadurch gekennzeichnet, daß man entweder
a) eine Verbindung der allgemeinen Formel IV

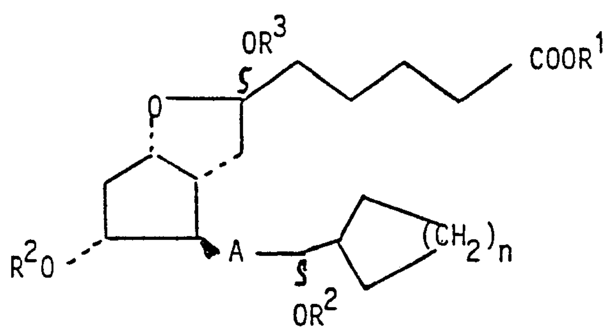

(IV),

worin
$R^1$, $R^2$, A und n die oben angegebene Bedeutung haben und
$R^3$ Wasserstoff, geradkettiges oder verzweigtes $C_{1-4}$-Alkyl bedeutet,
oxydiert und die erhaltene 7-Oxo-Verbindung der allgemeinen Formel II

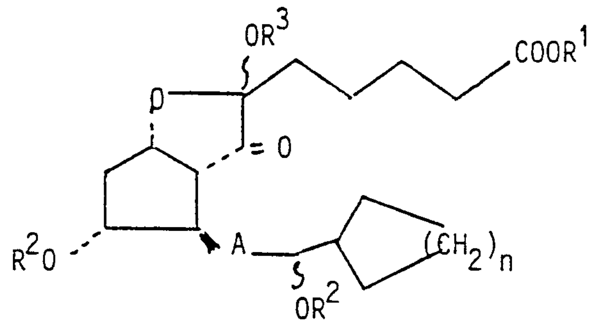

(II) ,

worin

$R^1$, $R^2$, $R^3$, A und n die oben angegebene Bedeutung haben,

erhitzt, oder

b) eine Verbindung der allgemeinen Formel III

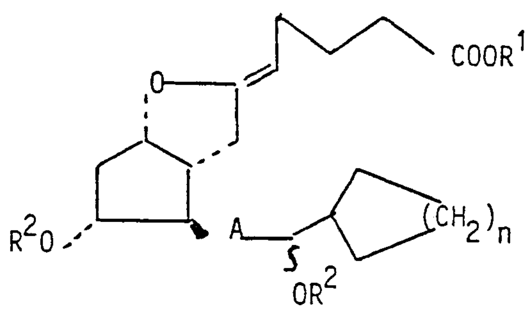

(III) ,

worin

$R^1$, $R^2$, A und n die oben angegebene Bedeutung haben,

oxydiert, wobei in den nach Variante a) oder Variante b) erhaltenen 7-Oxo-$PGI_2$-Derivaten der Formel I gewünschtenfalls die Schutzgruppen der 11- und/oder 15-Hydroxylgruppen und/oder der Carboxylgruppe entfernt und/oder die 7-Oxo-$PGI_2$-Derivate der Formel I in pharmakologisch anwendbare Salze übergeführt werden können.

7. Verfahren nach Anspruch 6, Variante a), dadurch gekennzeichnet, daß man die Oxydation der Verbindung der allgemeinen Formel IV in einem wasserhaltigen Lösungsmittel mit Selendioxyd durchführt.

8. Verfahren nach Anspruch 6, Variante a), dadurch gekennzeichnet, daß man das Erhitzen der 7-Oxo-Verbindung der allgemeinen Formel II in Anwesenheit eines Lösungsmittels in Gegenwart eines Säurekatalysators oder in Abwesenheit eines Lösungsmittels im Temperaturbereich von 50 - 200°C durchführt.

9. Verfahren nach Anspruch 6, Variante b), dadurch gekennzeichnet, daß man die Oxydation der Verbindung der allgemeinen Formel III in einem wasserfreien Lösungsmittel mit Selendioxyd durchführt.

10. Pharmazeutisches Präparat, das als Wirkstoff eine oder mehrere Verbindungen der Ansprüche 1 bis 5 enthält.

**Patentansprüche** für den Vertragsstaat: AT

1. Verfahren zur Herstellung von racemischen und optisch aktiven 7-Oxo-prostacyclin-Derivaten der allgemeinen Formel I

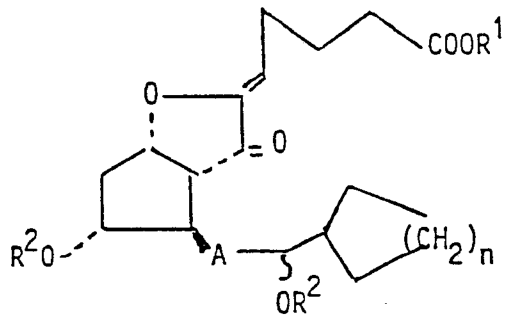

(I) ,

worin

$R^1$ Wasserstoff, ein geradkettiges oder verzweigtes $C_{1-4}$-Alkyl oder ein pharmakologisch anwendbares Kation,

$R^2$ Wasserstoff, $C_{1-4}$-Alkanoyl, Benzoyl, durch Halogen oder $C_{1-4}$-Alkyl substituiertes Benzoyl, Tetrahydropyranyl, Trialkylsilyl oder Alkoxy-alkyl,

A Äthylen, cis- oder trans-Vinylen oder -C≡C- und

n 2, 3 oder 4 bedeuten,

dadurch gekennzeichnet, daß man entweder

a) eine Verbindung der allgemeinen Formel IV

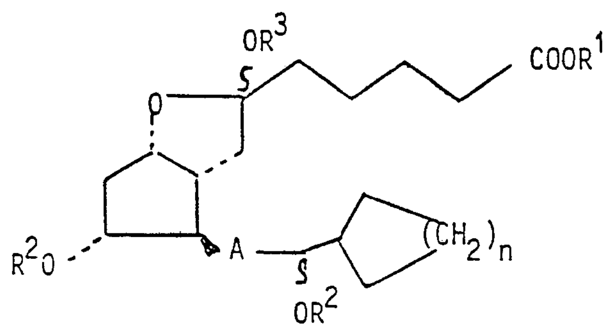

(IV),

worin

$R^1$, $R^2$, A und n die oben angegebene Bedeutung haben und

$R^3$ Wasserstoff, geradkettiges oder verzweigtes $C_{1-4}$-Alkyl bedeutet,

oxydiert und die erhaltene 7-Oxo-Verbindung der allgemeinen Formel II

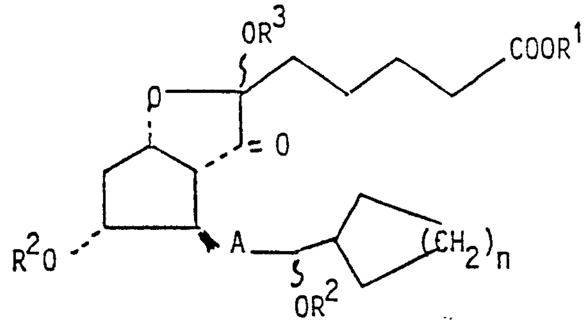

(II) ,

worin

$R^1$, $R^2$, $R^3$, A und n die oben angegebene Bedeutung haben,

erhitzt, oder

b) eine Verbindung der allgemeinen Formel III

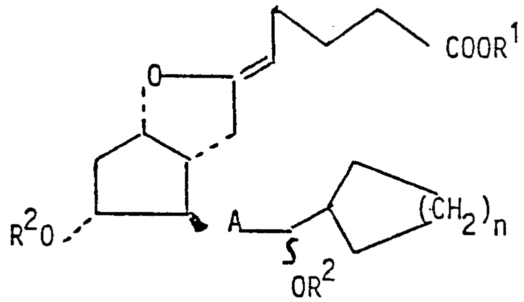

(III) ,

worin

$R^1$, $R^2$, A und n die oben angegebene Bedeutung haben,

oxydiert, wobei in den nach Variante a) oder Variante b) erhaltenen 7-Oxo-$PGI_2$-Derivaten der Formel I gewünschtenfalls die Schutzgruppen der 11- und/oder 15-Hydroxylgruppen und/oder der Carboxylgruppe entfernt und/oder die 7-Oxo-$PGI_2$-Derivate der Formel I in pharmakologisch anwendbare Salze übergeführt werden können.

2. Verfahren nach Anspruch 1, Variante a), dadurch gekennzeichnet, daß man die Oxydation der Verbindung der allgemeinen Formel IV in einem wasserhaltigen Lösungsmittel mit Selendioxyd durchführt.

3. Verfahren nach Anspruch 7, Variante a), dadurch gekennzeichnet, daß man das Erhitzen der 7-Oxo-Verbindung der allgemeinen Formel II in Anwesenheit eines Lösungsmittels in Gegenwart eines Säurekatalysators oder in Abwesenheit eines Lösungsmittels im Temperaturbereich von 50 - 200° C durchführt.

4. Verfahren nach Anspruch 1, Variante b), dadurch gekennzeichnet, daß man die Oxydation der Verbindung der allgemeinen Formel III in einem wasserfreien Lösungsmittel mit Selendioxyd durchführt.

5. Verwendung von einer oder mehreren der nach den verfahren der Ansprüche 1 bis 4 erhältlichen Verbindungen als Wirkstoffe zur Herstellung von pharmazeutischen Präparaten.

**Claims** for the Contracting States: BE, CH, DE, FR, GB, IT, LI, LU, NL, SE

1. Racemic and optically active 7-oxo-prostacycline derivatives of general formula I

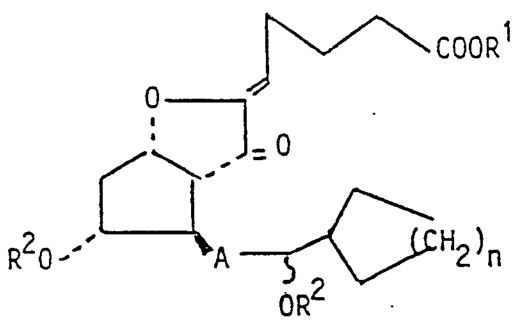

(I)

wherein

$R^1$ represents hydrogen, a straight-chained or branched $C_{1-4}$-alkyl or a pharmacologically usable cation,

$R^2$ represents hydrogen, $C_{1-4}$-alkanoyl, benzoyl, benzoyl substituted by halogen or $C_{1-4}$-alkyl, tetrahydropyranyl, trialkylsilyl or alkoxyalkyl,

A represents ethylene, cis- or trans-vinylene or -C≡C- and

n represents 2, 3 or 4.

2. 7-Oxo-11,15-diacetyl-16,17,18,19,20-pentanor-15-cyclopentyl-$PGI_2$-methyl ester.

3. Methyl ester, sodium salt or tris-(hydroxymethyl)-amino-methane salt of 7-oxo-16,17,18,19,20-pentanor-15-cyclopentyl-$PGI_2$.

4. 7-Oxo-11,15-diacetyl-16,17,18,19,20-pentanor-15-cyclohexyl-$PGI_2$-methyl ester.

5. 7-Oxo-16,17,18,19,20-pentanor-15-cyclohexyl-$PGI_2$-methyl ester.

6. Process for preparing racemic and optically active 7-oxo-prostacycline derivatives of general formula I

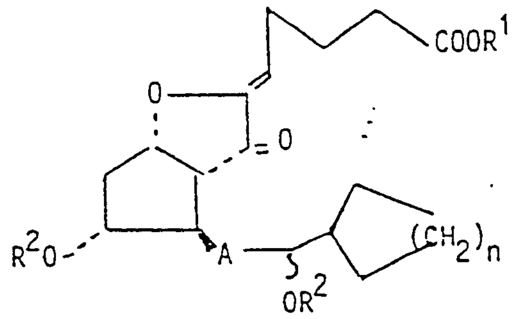

(I)

wherein
$R^1$ represents hydrogen, a straight-chained or branched $C_{1-4}$-alkyl or a pharmacologically usable cation,
$R^2$ represents hydrogen, $C_{1-4}$-alkanoyl, benzoyl, benzoyl substituted by halogen or $C_{1-4}$-alkyl, tetrahydropyranyl, trialkylsilyl or alkoxyalkyl,
A represents ethylene, cis- or trans-vinylene or -C≡C- and
n represents 2, 3 or 4,
characterised in that either
a) a compound of general formula IV

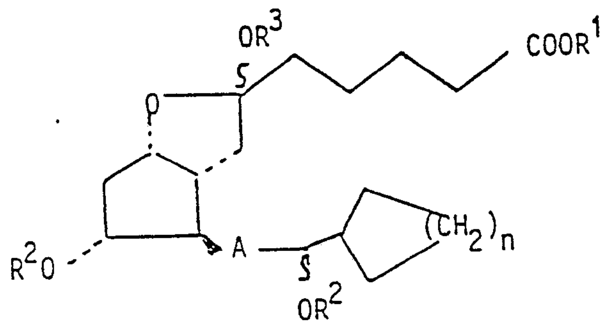

(IV),

wherein
$R^1$, $R^2$, A and n are as hereinbefore defined and
$R^3$ represents hydrogen, straight chained or branched $C_{1-4}$-alkyl,
is oxidised and the resulting 7-oxo compound of general formula II

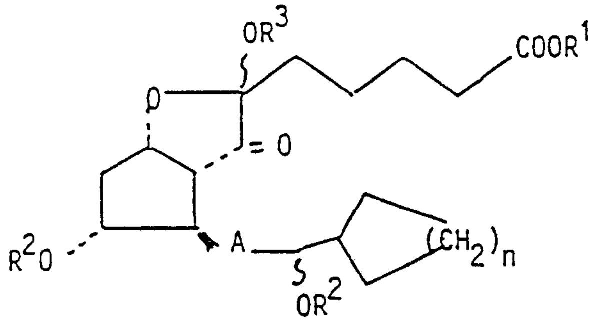

(II),

wherein
$R^1$, $R^2$, $R^3$, A and n are defined as hereinbefore,
is heated, or
b) a compound of general formula III

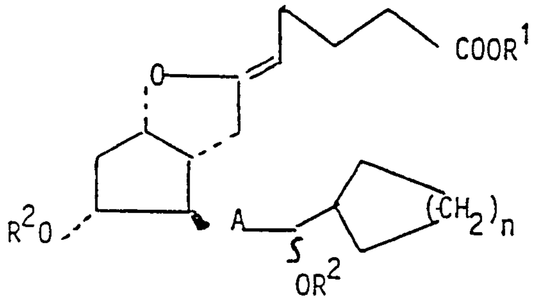

(III),

wherein

$R^1$, $R^2$, a and n are defined as hereinbefore,

is oxidised, whilst in the 7-oxo-$PGI_2$ derivatives of formula I obtained according to variant a) or variant b) the protective groups of the 11- and/or 15-hydroxyl groups and/or of the carboxyl group are removed if desired and/or the 7-oxo-$PGI_2$ derivatives of formula I may be converted into pharmacologically usable salts.

7. Process as claimed in claim 6, variant a), characterised in that the oxidation of the compound of general formula IV is carried out in an aqueous solvent with selenium dioxide.

8. Process as claimed in claim 6, variant a), characterised in that the heating of the 7-oxo compound of general formula II is carried out in the presence of a solvent in the presence of an acid catalyst or in the absence of a solvent in the temperature range from 50 to 200°C.

9. Process as claimed in claim 6, variant b), characterised in that the oxidation of the compound of general formula III is carried out in an aqueous solvent with selenium dioxide.

10. Pharmaceutical preparation containing as active substance one or more compounds of claims 1 to 5.

**Claims** for the Contracting State: AT

1. Process for preparing racemic and optically active 7-oxo-prostacycline derivatives of general formula I

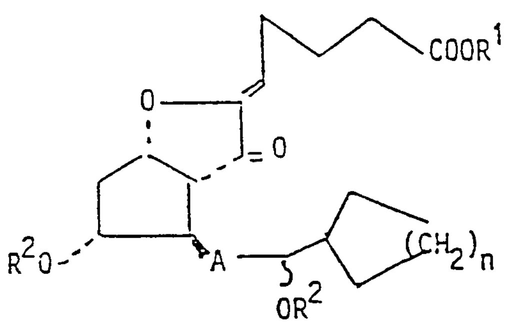

(I)

wherein

$R^1$ represents hydrogen, a straight-chained or branched $C_{1-4}$-alkyl or a pharmacologically usable cation,

$R^2$ represents hydrogen, $C_{1-4}$-alkanoyl, benzoyl, benzoyl substituted by halogen or $C_{1-4}$-alkyl, tetrahydropyranyl, trialkylsilyl or alkoxyalkyl,

A represents ethylene, cis- or trans-vinylene or -C≡C- and

n represents 2, 3 or 4,

characterised in that either

a) a compound of general formula IV

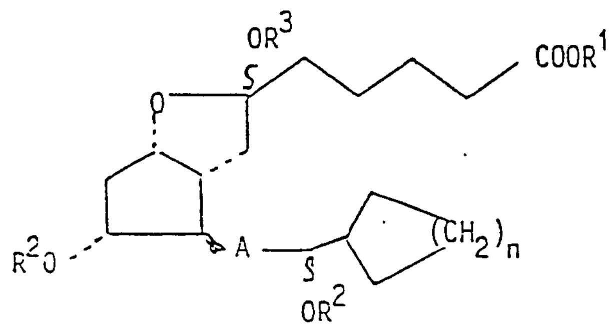

(IV),

wherein
$R^1$, $R^2$, A and n are as hereinbefore defined and
$R^3$ represents hydrogen, straight chained or branched $C_{1-4}$-alkyl,
is oxidised and the resulting 7-oxo compound of general formula II

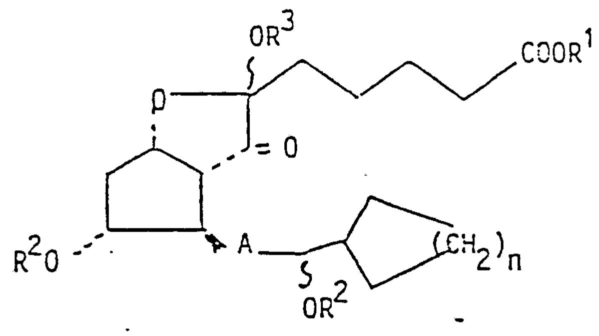

(II),

wherein
$R^1$, $R^2$, $R^3$, A and n are defined as hereinbefore,
is heated, or
b) a compound of general formula III

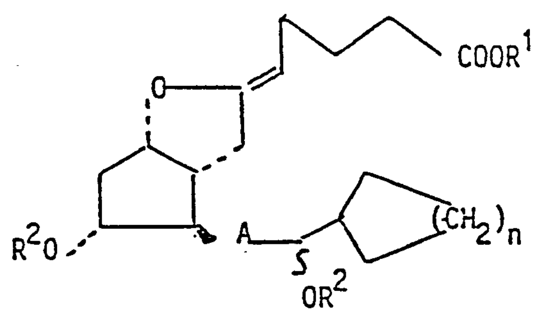

(III),

wherein
$R^1$, $R^2$, A and n are defined as hereinbefore,
is oxidised, whilst in the 7-oxo-$PGI_2$ derivatives of formula 1 obtained according to variant a) or variant b) the protective groups of the 11- and/or 15-hydroxyl groups and/or of the carboxyl group are removed if desired and/or the 7-oxo-$PGI_2$ derivatives of formula I may be converted into pharmacologically usable salts.

2. Process as claimed in claim 1, variant a), characterised in that the oxidation of the compound of general formula IV is carried out in an aqueous solvent with selenium dioxide.

3. Process as claimed in claim 1, variant a), characterised in that the heating of the 7-oxo compound of general formula II is carried out in the presence of a solvent in the presence of an acid catalyst or in the absence of a solvent in the temperature range from 50 to 200° C.

4. Process as claimed in claim 1, variant b), characterised in that the oxidation of the compound of general formula III is carried out in an aqueous solvent with selenium dioxide.

5. Use of one or more of the compounds obtainable according to the processes of claims 1 to 4 as active substances for the production of pharmaceutical preparations.

**Revendications** pour les Etats Contractants: BE, CH, DE, FR, GB, IT, LI, LU, NL, SE

1. Dérivés de 7-oxo-prostacycline racémiques et optiquement actifs de formule générale I

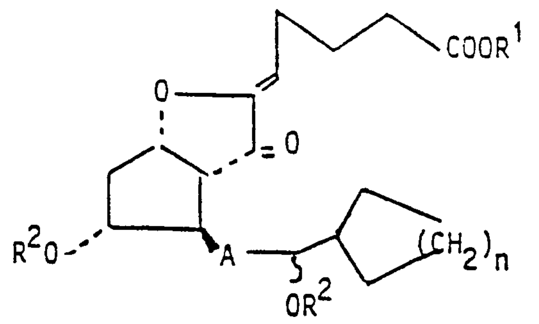

(I),

dans laquelle
$R^1$ est l'hydrogène, un alkyle $C_{1-4}$ linéaire ou ramifié ou un cation pharmacologiquement utilisable,
$R^2$ est l'hydrogène, un alcanoyle $C_{1-4}$, benzoyle, benzoyle substitué par un halogène ou un alkyle $C_{1-4}$, tétrahydropyrannyle, trialkylsilyle ou alcoxyalkyle,
A est l'éthylène, le cis- ou trans-vinylène ou -C≡C- et
n est 2, 3 ou 4.

2. Ester méthylique de 7-oxo-11,15-diacétyl-16,17,18,19,20-pentanor-15-cyclopentyl-$PCl_2$.

3. Ester méthylique, sel de sodium ou sel de tris-(hydroxyméthyl)-aminométhane de 7-oxo-16,17,18,19,20-pentanor-15-cyclopentyl-$PGI_2$.

4. Ester méthylique de 7-oxo-11,15-diacétyl-16,17,18,19,20-pentanor-15-cyclohexyl-$PGI_2$.

5. Ester méthylique de 7-oxo-16,17,18,19,20-pentanor-15-cyclohexyl-$PGI_2$.

6. Procédé de préparation de dérivés de 7-oxo-prostacycline racémique et optiquement actifs de formule générale I

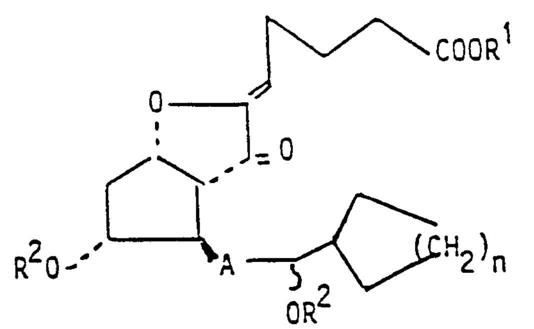

(I),

dans laquelle
$R^1$ est l'hydrogène, un alkyle $C_{1-4}$ linéaire ou ramifié ou un cation pharmacologiquement utilisable,
$R^2$ est l'hydrogène, un alcanoyle $C_{1-4}$, benzoyle, benzoyle substitué par un halogène ou un alkyle $C_{1-4}$, tétrahydropyrannyle, trialkylsilyle ou alcoxyalkyle,
A est l'éthylène, le cis- ou le trans-vinylène ou -C≡C- et
n est 2, 3 ou 4,
caractérisé en ce que, soit
a) on oxyde un composé de formule générale IV

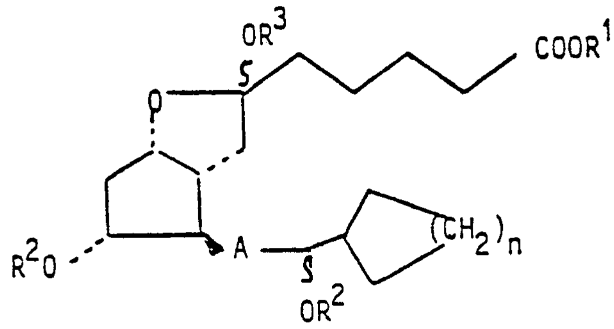

(IV),

dans laquelle
$R^1$, $R^2$, A et n ont la signification donnée ci-dessus, et
$R^3$ est l'hydrogène, un alkyle linéaire ou ramifié $C_{1-4}$,
et on chauffe le composé 7-oxo obtenu de formule générale II

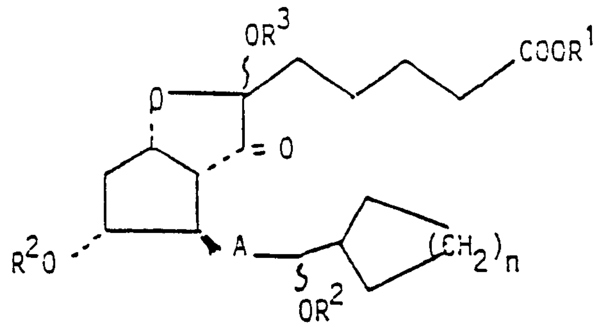

(II),

dans laquelle
$R^1$, $R^2$, $R^3$, A et n ont la signification donnée ci-dessus, soit
b) on oxyde un composé de formule générale III

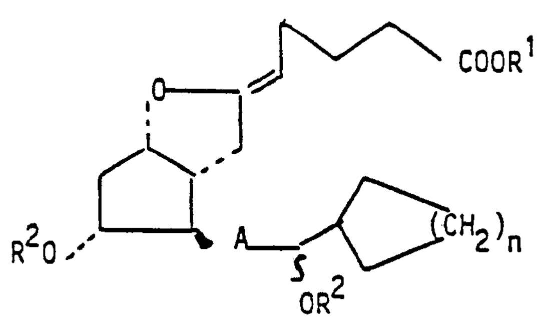

(III),

dans laquelle
$R^1$, $R^2$, A et n ont la signification donnée ci-dessus,
en éliminant des dérivés 7-oxo-$PGI_2$ de formule I obtenus selon la variante a) ou la variante b), si c'est souhaité, les groupes protecteurs des groupes hydroxyle 11 et/ou 15 et/ou du groupe carboxyle et/ou on peut transformer les dérivés 7-oxo-$PGI_2$ en sels pharmacologiquement compatibles.

7. Procédé selon la revendication 6, variante a), caractérisé en ce qu'on réalise l'oxydation du composé de formule générale IV dans un solvant contenant de l'eau, avec de l'oxyde de sélénium.

8. Procédé selon la revendication 6, variante a), caractérisé en ce qu'on réalise le chauffage du composé 7-oxo de formule générale II en présence d'un solvant et d'un catalyseur acide, ou sans solvant dans un intervalle de température de 50 - 200°C.

9. Procédé selon la revendication 6, variante b), caractérisé en ce qu'on réalise l'oxydation du composé de formule générale III dans un solvant anhydre, avec de l'oxyde de sélénium.

10. Préparation pharmaceutique, qui contient comme principe actif un ou plusieurs composés des revendications 1 à 5.

**Revendications** pour l'Etat Contractant: AT

1. Procédé de preparation de dérivés de 7-oxo-prostacycline racémiques et optiquement actifs de formule générale I

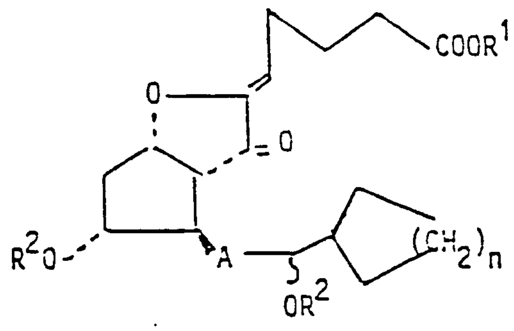

(I),

dans laquelle
$R^1$ est l'hydrogène, un alkyle $C_{1-4}$ linéaire ou ramifié ou un cation pharmacologiquement utilisable,
$R^2$ est l'hydrogène, un alcanoyle $C_{1-4}$, benzoyle, benzoyle substitué par un halogène ou un alkyle $C_{1-4}$, tétrahydropyrannyle, trialkylsilyle ou alcoxyalkyle,
A est l'éthylène, le cis- ou trans-vinylène ou -C≡C- et
n est 2, 3 ou 4,
caractérisé en ce que, soit
a) on oxyde un composé de formule générale IV

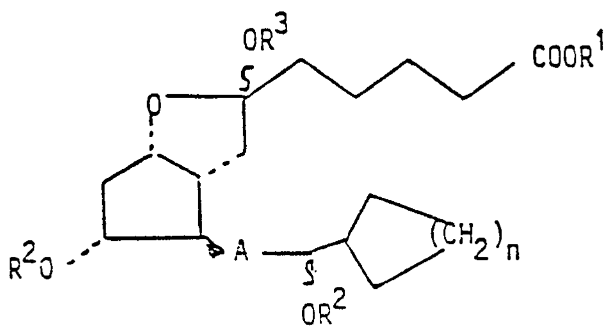

(IV),

dans laquelle
$R^1$, $R^2$, A et n ont la signification donnée ci-dessus, et
$R^3$ est l'hydrogène, un alkyle linéaire ou ramifié $C_{1-4}$,
et on chauffe le composé 7-oxo obtenu de formule générale II

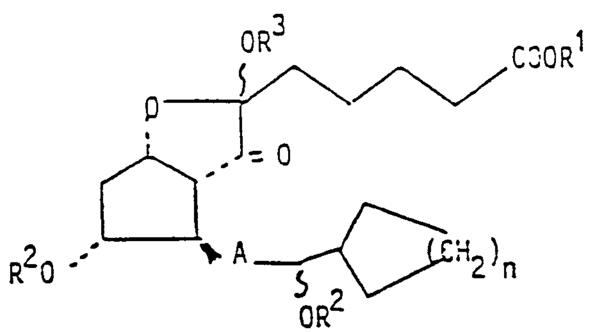

(II),

dans laquelle $R^1$ $R^2$ $R^3$ A et n ont la signification donnée ci-dessus, soit
b) on oxyde un composé de formule générale III

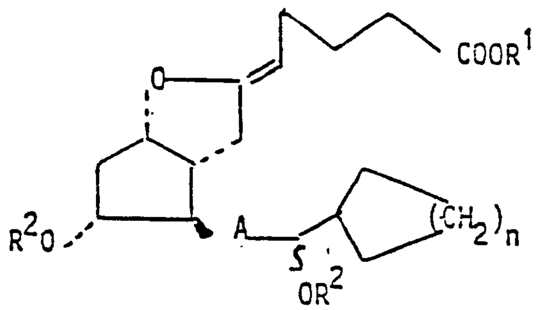

(III),

dans laquelle

$R^1$, $R^2$, A et n ont la signification donnée ci-dessus,

en éliminant des dérivés 7-oxo-$PGI_2$ de formule I obtenus selon la variante a) ou la variante b), si c'est souhaité, les groupes protecteurs des groupes hydroxyle 11 et/ou 15 et/ou du groupe carboxyle et/ou on peut transformer les dérivés 7-oxo-$PGI_2$ en sels pharmacologiquement compatibles.

2. Procédé selon la revendication 1, variante a), caractérisé en ce qu'on réalise l'oxydation du composé de formule générale IV dans un solvant contenant de l'eau, avec de l'oxyde de sélénium.

3. Procédé selon la revendication 1, variante a), caractérisé en ce qu'on réalise le chauffage du composé 7-oxo de formule générale II en présence d'un solvant et d'un catalyseur acide, ou sans solvant dans un intervalle de température de 50 - 200° C.

4. Procédé selon la revendication 1, variante b), caractérisé en ce qu'on réalise l'oxydation du composé de formule générale III dans un solvant anhydre, avec de l'oxyde de sélénium.

5. Utilisation d'un ou plusieurs composés obtenus selon les procédés des revendications 1 à 4 comme principes actifs pour fabriquer des préparations pharmaceutiques.